(19) Europäisches Patentamt
European Patent Office
Office européen des brevets

(11) Numéro de publication: **0 188 154**
**B1**

(12) **FASCICULE DE BREVET EUROPÉEN**

(45) Date de publication du fascicule du brevet: **04.11.87**

(51) Int. Cl.⁴: **C 07 C 43/225**, C 07 C 41/01

(21) Numéro de dépôt: **85402485.8**

(22) Date de dépôt: **13.12.85**

(54) **Procédé d'estérification et de fluoration simultanées.**

(30) Priorité: **26.12.84 FR 8419798**

(43) Date de publication de la demande: **23.07.86 Bulletin 86/30**

(45) Mention de la délivrance du brevet: **04.11.87 Bulletin 87/45**

(84) Etats contractants désignés: **AT BE CH DE FR GB IT LI LU NL SE**

(56) Documents cité: **CHEMICAL ABSTRACTS, vol. 83, no. 21, 24 novembre 1975, page 521, abrégé no. 178464f, Columbus, Ohio, US; V.M. BELOUS et al.: "Fluorination of aryl esters of trifluoroacetic acid by sulfur tetrafluoride in a hydrogen fluoride solution"**

(73) Titulaire: **RHONE- POULENC CHIMIE, 25, quai Paul Doumer, F-92408 Courbevoie Cédex (FR)**

(72) Inventeur: **Desbois, Michel, 526, Chemin du Bois, F-69140 Rillieux (FR)**

(74) Mandataire: **Cazes, Jean- Marie, RHONE- POULENC INTERSERVICES Service Brevets Chimie Centre de Recherches de Saint- Fons B.P. 62, F-69192 Saint- Fons Cédex (FR)**

**EP 0 188 154 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

## Description

La présente invention concerne un procédé de préparation de composés porteurs d'un groupe difluorométhylène situé en α d'un atome d'oxygène. Cette invention concerne plus particulièrement un procédé d'estérification et de fluoration simultanées permettant la préparation de composés porteurs d'un groupe difluorométhylène en de l'oxygène.

Il est connu d'après le brevet anglais N° 1 077 547 d'estérifier des diacides gras par un alcool dans un milieu constitué d'acide fluorhydrique liquide. L'ester est obtenu avec de bons rendements mais on n'obtient aucune trace de dérivés contenant un groupe diflourométhylène en α de l'oxygène.

Il est aussi connu d'après Topchiev dans "Boron fluoride and its compounds as catalysts in organic chemistry" de l'Académie des Sciences d'URSS (1959, Pergamon Press, pages 267-269) de préparer des esters par condensation d'acides carboxyliques et d'alcools en présence de trifluorure de bore. L'ester est obtenu avec des rendements variables selon la nature de l'acide mais aucune trace de composés porteurs d'un groupe difluorométhylènoxy n'est obtenue.

L'art antérieur ne suggère aucune solution au problème d'obtention de dérivés porteurs de groupe difluorométhylènoxy à partir des acides et des alcools correspondants.

La présente invention a su atteindre cet objectif et a pour objet un procédé de préparation de composés porteurs d'un groupe difluorométhylènoxy par estérification et fluoration simultanées, caractérisé en ce qu'on met en contact dans l'acide fluorhydrique liquide anhydre, un alcool ou un phénol avec un composé choisi parmi l'acide trifluoroacétique, ses halogénures et son anhydride, en présence de trifluorure de bore en quantité telle que la pression absolue de trifluorure de bore soit d'au moins un bar.

L'alcool mis en oeuvre peut être un alcool aliphatique ou aromatique, un phénol ou un dérivé phénolique. On entend par dérivé phénolique, tout phénol substitué sur le noyau aromatique par un halogène, un groupe alkyle, alcoxy, thioalkyle, phényle, phénoxy, nitro, amino, carbonyle.

Le rapport molaire de l'acide trifluoracétique, de son halogénure ou de son anhydride au phénol ou à l'alcool est de préférence compris entre 0,5 et 2 et encore plus préférentiellement d'au moins un.

Il est préférable de travailler avec une quantité de $BF_3$ telle que la pression absolue de $BF_3$ dans l'encainte réactionnalle soit comprise entre 5 et 50 bars.

Avantageusement, le rapport molaire de l'acide fluorhydrique à l'acide trifluoracétique ou à son dérivé est compris entre 5 et 50. Encore plus préférentrellemant, ce rapport est compris entre 10 et 30.

La température de la réaction est de préférence comprise entre 0 et 150° C et encore plus préférentiellement entre 20 et 80° C.

La réaction peut avoir lieu en présence de solvants. Parmi les solvants utilisables, on peut citer, mais non limitativement: $CCl_4$, $CHCL_3$, $CFCl_2$-$CF_2Cl$.

Parmi les composés obtenus selon le procédé de l'invention, on peut citer les produits suivants:

pentafluoroéthoxybenzène, chloro-4 pentafluoroéthoxybenzène, difluoro-α,α trichloro-β,β,β éthoxybenzène, nitro-4 pentafluoroéthoxybenzène, phénoxy-4 pentafluoroéthoxybenzène, fluoro-4 pentafluoroéthoxybenzène, trifluorométhyl-3 pentafluoroéthoxybenzène, trifluorométhyl-4 pentafluoroéthoxybenzène, chloro-2 trifluorométhyl-4 pentafluoroéthoxybenzène, trifluorométhoxy-4 pentafluoroéthoxybenzène, pentafluoroéthyltrifluoro-β,β,β éthyléther.

Les composés obtenus par le procédé de l'invention sont utilisés comme intermédiaires de synthèse dans l'industrie pharmaceutique ou phytosanitaire, comme anesthésiques (Kirk Othmer II, p. 684-689) et additifs pour huiles lubrifiantes.

L'invention sera plus explicitement décrite sans toutefois en limiter la portée à l'aide des exemples qui vont suivre.

### Exemple 1

Dans un réacteur inox de 250 ml, on introduit successivement 50 g (2,5 m) d'HF anhydre, 12,9 g (0,1 m) de p-chlorophénol et 22,8 g (0,2 m) d'acide trifluoroacétique. Le réacteur est fermé, porté à la précision de 20 bars à 0° C par du $BF_3$ gazeux puis chauffé 18 heures à 70° C. Après refroidissement et décompression, le mélange brut acide est coulé sur 200 g de glace pilée puis extrait par 3 fois 100 ml de $CH_2Cl_2$. Lea phases organiques sont rassemblées, lavées par 100 ml d'eau permutée et séchées. Par analyses chromatographie phase gazeuse et spectrométrie de masse, on obtient la composition suivante:

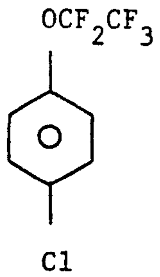 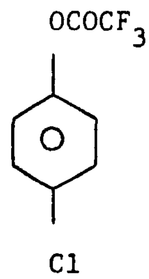 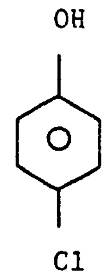

| | | |
|---|---|---|
| 8,1 % | 49 % | 42,6 % |

**Exemple comparatif 1A**

On opère de la même façon qu'à l'exemple 1 en mettant en oeuvre :
- 12,9 g (0,1 m) de parachlorophénol
- 57 g (0,5 m) d'acide trifluoracétique

On pressurise le réacteur avec 19 bars de $BF_3$ à 14°C et on chauffe à 80°C pendant 2 H 30 à une pression maximale de 24 bars. Après analyse chromatographie phase gazeuse couplée avec la spectrométrie de masse, on obtient : 79,8 % de trifluoroacétate de p-chlorophényle et 20,2 % de parachlorophénol et aucune trace de parachloropentafluoroéthoxybenzène n'est mise en évidence.

**Exemple comparatif 1B**

On opère de la même façon qu'à l'exemple 1 en mettant en oeuvre:
- 12,9 g (0,1 m) de p-chlorophénol
- 22,8 g (0,2 m) d'acide trifluoracétique
- 100 g (5 m) d'acide fluorhydrique anhydre

On chauffe à 80°C pendant 23 H 30 sous une pression maximale de 5 bars. Après analyse chromatographie phase gazeuse couplée avec la spectrométrie de masse, on obtient 26,9 % de trifluoroacétate de p-chlorophényle et 66,7 % de parachlorophénol et aucune trace de parachloropentafluoroéthoxybenzène n'est mise en évidence.

**Exemple 2**

On opère de la même façon qu'à l'exemple 1 avec les produits et conditions suivants:

| | |
|---|---|
| 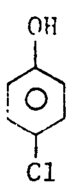 | 0,1 mole = 12,9 g |
| $(CF_3CO)_2O$ | 0,1 mole = 21 g |
| HF | 2,5 moles = 50 g |
| $BF_3$ | 25 bars à 3°C |
| T° | 20°C |
| Durée | 18 H 30 |

Après traitement, les analyses par couplage chromatographie phase gazeuse, spectrométrie de masse nous montrent la présence de chloro-4 pentafluoroéthoxybenzène.

**Exemple 3**

On opère de la même façon qu'à l'exemple 1 avec les produits et conditions suivants:

| | |
|---|---|
| 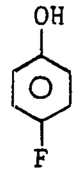 | 0,3 mole = 33,6 g |
| $CF_3COOH$ | 0,3 mole = 34,2 g |
| HF | 5 moles = 100 g |
| $BF_3$ | 30 bars à 20°C |
| T° | 50°C |
| Durée | 8 heures |

Après traitements, les analyses par couplage cbromatographie phase gazeuse, spectrométrie de masse nous montrent la présence de fluoro-4 pentafluoroéthoxybenzène.

**Exemple 4**

On opère de la même façon qu'à l'exemple 1 avec les produits et conditions suivants:

| | |
|---|---|
| 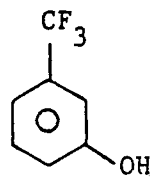 | 0,2 mole = 32,4 g |
| $CF_3COOH$ | 0,3 mole |
| HF | 5 moles = 100 g |
| $BF_3$ | 50 bars à 10°C |
| T° | 20°C |
| Durée | 22 heures |

Après traitement, les analyses par couplage chromatographie phase gazeuse, spectrométrie de masse nous montrent la présence de trifluorométhyl-3 pentafluoroéthoxybenzène.

**Revendications**

1. Procédé de préparation de composés porteurs d'un groupe difluorométhylènoxy par estérification et fluoration simultanées caractérisé en ce qu'on met en contact dans l'acide fluorhydrique liquide anhydre, un alcool ou un phénol avec un composé choisi parmi l'acide trifluoroacétique, ses halogénures et son anhydride en présence de trifluorure de bore en quantité telle que la pression absolue de trifluorure de bore soit d'au moins un bar.

2. Procédé selon la revendication 1 caractérisé en ce qu'on utilise un rapport molaire d'acide fluorhydrique à l'acide trifluoracétique, à son halogénure ou à son anhydride compris entre 5 et 50 et de préférence entre 10 et 30.

3. Procédé selon la revendication 1 caractérisé en ce qu'on utilise du trifluorure de bore à une pression comprise entre 5 et 50 bars.

4. Procédé selon la revendication 1 caractérisé en ce que la température de réaction est comprise entre 0 et 150°C et de préférence entre 20 et 80°C.

5. Procédé selon la revendication 1 caractérisé en ce qu'on ajoute un solvant.
6. Procédé selon la revendication 5 caractérisé en ce que le solvant est choisi parmi $CCl_4$, $CHCl_3$, $CFCl_2$-$CClF_2$.

**Patentansprüche**

1. Verfahren zur Herstellung von Verbindungen, die eine Difluormethylenoxygruppe tragen, durch gleichzeitige Veresterung und Fluorierung, dadurch gekennzeichnet, daß man in wasserfreier flüssiger Fluorwasserstoffsäure einen Alkohol oder ein Phenol mit einer Verbindung ausgewählt aus Trifluoressigsäure, ihren Halogeniden und ihrem Anhydrid in Kontakt bringt, in Gegenwart einer solchen Menge Bortrifluorid, daß der absolute Druck des Bortrifluorids mindestens ein bar beträgt.
2. Verfahren nach Anspruch 1, dadurch gekennzeichnet, daß man ein molares Verhältnis von Fluorwasserstoffsäure zu Trifluoressigsäure, ihrem Halogenid oder ihrem Anhydrid von zwischen 5 und 50, vorzugsweise zwischen 10 und 30 einsetzt.
3. Verfahren nach Anspruch 1, dadurch gekennzeichnet, daß man Bortrifluorid unter einem Druck von zwischen 5 und 50 bar einsetzt.
4. Verfahren nach Anspruch 1, dadurch gekennzeichnet, daß die Reaktionstemperatur zwischen 0 und 150 C, vorzugsweise zwischen 20 und 80° C liegt.
5. Verfahren nach Anspruch 1, dadurch gekennzeichnet, daß man ein Solvens zugibt.
6. Verfahren nach Anspruch 5, dadurch gekennzeichnet, daß das Solvens ausgewählt wird aus $CCl_4$, $CHCl_3$, $CFCl_2$-$CClF_2$.

**Claims**

1. Process for the preparation of compounds carrying a difluoromethyleneoxy group by simultaneous esterification and fluorination, characterized in that an alcohol or a phenol is brought into contact, in anhydrous liquid hydrofluoric acid, with a compound chosen from among trifluoroacetic acid, its halides and its anhydride, in the presence of boron trifluoride in such amount that the absolute pressure of boron trifluoride is at least one bar.
2. Process according to Claim 1, characterized in that a molar ratio of hydrofluoric acid to trifluoroacetic acid, its halide or its anhydride of between 5 and 50, and preferably of between 10 and 30, is used.
3. Process according to Claim 1, characterized in that boron trifluoride is used at a pressure of between 5 and 50 bars.
4. Process according to Claim 1, characterized in that the reaction temperature is between 0 and 150° C and preferably between 20 and 80° C.
5. Process according to Claim 1, characterized in that a solvent is added.
6. Process according to Claim 5, characterized in that the solvent is chosen from amongst $CCl_4$, $CHCl_3$ and $CFCl_2$-$CClF_2$.